# EUROPEAN PATENT APPLICATION

(11) **EP 4 652 864 A1**
(43) Date of publication of application: **26.11.2025**
(21) Application number: 24177538.6
(22) Date of filing: 23.05.2024
(51) Int. Cl.: A24F 40/05, A24F 40/42, A24F 40/10, A24F 40/30, A61M 11/00

(54) **AEROSOL GENERATING APPARATUS**

(71) Applicant: Imperial Tobacco Limited, Bristol, BS3 2LL (GB)
(72) Inventor: The designation of the inventor has not yet been filed
(74) Representative: Haseltine Lake Kempner LLP

(57) **Abstract**

The invention provides replaceable cartridge for an aerosol generating device, the replaceable cartridge comprising: a tank for containing a liquid aerosol precursor; an aerosol generating unit for generating an aerosol from the liquid aerosol precursor, wherein the aerosol generating unit comprises a piezoelectric transducer; a cartridge memory unit adapted to store an inhalation metric associated with the aerosol generating unit; and a cartridge communication unit adapted to: receive an inhalation metric update from the aerosol generating device for updating the inhalation metric stored in the cartridge memory unit.

## Description

### FIELD

The present disclosure relates to an aerosol generating apparatus.

### BACKGROUND

A typical aerosol generating apparatus may comprise a power supply, an aerosol generating unit that is driven by the power supply, an aerosol precursor, which in use is aerosolised by the aerosol generating unit to generate an aerosol, and a delivery system for delivery of the aerosol to a user.

In some cases, the aerosol generating unit may include an ultrasonic generator e.g. a piezoelectric transducer (PET) for generating the aerosol. In use, the surface of the PET will expand and contract as it vibrates.

A PET generates aerosol by causing cavitation to occur within a liquid aerosol precursor that is provided on a surface of the PET. Cavitation refers to the phenomenon where the static pressure of a liquid reduces to below the liquid's vapour pressure, leading to the formation of small vapour filled cavities within the liquid. When the cavities are subsequently subjected to a higher pressure, the cavities collapse resulting in a shock wave that propagates through the liquid. This shock wave induces capillary waves, or ripples, in a surface distal (referred to herein as the upper surface of the liquid) from the PET that may form ligaments to expel droplets from the upper surface. More succinctly, in a thin layer of liquid, the collapsing of the cavities can induce a disturbance in the liquid that causes liquid droplets to be expelled from liquid, thereby forming an aerosol over the surface of the liquid, typically within an aerosolisation chamber.

In the context of a PET for generating the aerosol, when the surface of the PET expands, the liquid on the surface will conform to the expanded surface. When the surface of the PET subsequently contracts, the static pressure in the liquid will fall as it is effectively dragged with the surface with the decrease in static pressure being proportional to the speed of the movement of the PET surface (i.e., the frequency of the vibration). If the frequency and the amplitude of vibration of the PET are sufficiently high, cavitation will occur as a result of the contraction.

When the surface of the PET subsequently expands, the static pressure in the liquid will rise as it is effectively compressed by the surface with the increase in static pressure being proportional to the speed of the movement of the PET surface (i.e., the frequency of the vibration). Any cavities in the liquid previously formed may then implode, generating shock waves in the liquid capable of expelling droplets to form an aerosol.

In an aerosol generating apparatus using a PET, a liquid aerosol precursor is typically applied to the PET surface using a wick in fluid communication with a tank. The aerosol generated by cavitation of the liquid aerosol precursor will be drawn from the aerosolisation chamber along an aerosol flow path by suction at a mouthpiece outlet.

Aerosol generating apparatuses that use a PET for generating the aerosol present numerous challenges, including accurately driving the vibrational element and inefficiencies in the requisite circuitry.

In spite of the effort already invested in the development of aerosol generating apparatuses/systems further improvements are desirable.

### SUMMARY

In a first aspect the present disclosure provides replaceable cartridge for an aerosol generating device that comprises a tank for containing a liquid aerosol precursor and an aerosol generating unit for generating an aerosol from the liquid aerosol precursor.

In some examples, the replaceable cartridge further comprises a cartridge memory unit adapted to store a set of one or more driving parameters of the aerosol generating unit.

In some examples, the replaceable cartridge further comprises a cartridge communication unit adapted to communicate the set of one or more driving parameters to the aerosol generating device for driving the aerosol generating unit.

In other words, there is provided a replaceable cartridge with a memory unit for receiving and storing driving parameters for driving the aerosol generating unit in the cartridge.

Put another way, the driving parameters for driving an aerosol generating unit may be stored locally within a memory on the cartridge itself.

By providing an on-cartridge memory unit the invention provides a means of storing the driving parameters for driving and aerosol generating unit in the same, removable, part of the system as the aerosol generating unit itself. In this way, should the replaceable cartridge be separated from a given aerosol generating device and inserted into a different aerosol generating device, the aerosol generating unit can still be driven using the same stored driving parameters in order to maintain consistent aerosol delivery from the replaceable cartridge.

In some examples, the aerosol generating unit comprises a piezoelectric transducer for converting a driving electrical signal into movement. Piezoelectric transducers require a combination of interrelated driving parameters in order to be driven at an optimal efficiency. Each of the driving parameters has an effect on each of the other driving parameters, meaning that tuning a driving signal for driving a piezoelectric transducer in an optimal manner is a complex task as an adjustment to any of the driving parameters will have a knock-on effect on the remaining driving parameters. By storing the driving parameters for a given piezoelectric transducer locally to, i.e., in the same replaceable cartridge as, the piezoelectric transducer, it may be ensured that the piezoelectric transducer is driven at an optimal efficiency even if the replaceable cartridge is used with a different or new aerosol generating device.

In some examples, the set of one or more driving parameters comprises a driving frequency and a driving duty cycle.

The driving frequency at which the piezoelectric transducer is driven affects one or more properties of the aerosol generated by the aerosol generating apparatus - e.g., according to a user's preference, manufacturer/provider's recommendation, and/or regulatory requirement. Adjusting the driving frequency may be particularly suitable for adjusting an average size of droplets of the aerosol precursor entrained in the generated aerosol and/or a distribution of the size of said droplets. The inventors have observed that the frequency of the driving signal is a parameter that directly affects the average size of droplets in the aerosol generated by the aerosol generating apparatuses described herein. In particular, when driving the piezoelectric transducer with a driving signal having a relatively higher driving frequency, a relatively smaller average droplet size is observed. This observation is consistent with the physical mechanism for cavitation described in Kooij et al. Sci. Rep., 9, 6128 (2019), the entirety of which is incorporated herein by reference.

The driving frequency of a piezoelectric transducer has a direct effect on the efficiency of the driving of the piezoelectric transducer. In particular, the most efficient driving point of a given piezoelectric transducer is the resonant frequency of the piezoelectric transducer. Resonant frequencies are understood to be the frequency where a medium, such as a piezoelectric crystal within a piezoelectric transducer, vibrates at the highest amplitude for a given input power.

Thus, in the context of an aerosol generating device, a piezoelectric transducer driven with a driving frequency equal to the resonant frequency of the piezoelectric transducer will produce more aerosol than a piezoelectric transducer driven with a driving frequency not equal to the resonant frequency of the piezoelectric transducer for the same input power.

By storing the driving frequency, and in particular an optimal driving frequency, locally to the piezoelectric transducer it may be ensured that the piezoelectric transducer is driven at the optimal driving frequency, such as the resonant frequency of the piezoelectric transducer, rather than having to identify the optimal driving frequency of the piezoelectric transducer each time it is driven or provided to a new aerosol generating device.

During the lifetime of a piezoelectric transducer, the resonant frequency of said piezoelectric transducer may shift over time. Such a shift in resonant frequency may occur on a short-term scale, for example due to the piezoelectric transducer heating up during a single usage session, and may also occur on a long-term scale, for example where the resting resonance frequency (i.e., the resonance frequency of the piezoelectric transducer when it is not in use) changes over time due to material fatigue.

Accordingly, the invention also provides a means of storing a tracked change in a resonant frequency of a piezoelectric transducer and for use in driving the piezoelectric transducer at the changed resonant frequency. In this way, the aerosol generating device may be adapted to produce the target response of the piezoelectric transducer to the driving signal even as the optimal driving parameters for a given piezoelectric transducer change over time.

The driving duty cycle at which the driving signal drives the piezoelectric transducer affects one or more properties of the aerosol generated by the aerosol generating apparatus - e.g., according to a user's preference, manufacturer/provider's recommendation, and/or regulatory requirement. Adjusting the driving duty cycle may be particularly suitable for adjusting an amount of aerosol generated by a piezoelectric transducer.

The duty cycle of a signal is understood to be the fraction of one period in which a signal is active, the period being the time period for a signal to complete an on-off cycle. For example, a driving duty cycle of 50% will have a portion of the period where the driving signal is active, and is being applied to the piezoelectric transducer, and an equal period where the driving signal is inactive, an no signal is being applied to the piezoelectric transducer.

Conventionally, it has been assumed that a higher driving duty cycle would result in a larger amount of aerosol being generated by a piezoelectric transducer. However, the inventors have observed that this is not necessarily the case.

The driving signal may be a direct current signal such that the piezoelectric transducer receives a signal having a single polarity.

Upon application of a current in a first polarity, opposite faces of the piezoelectric crystal of a piezoelectric transducer respond by expanding, or bulging, outwards to define respective convex surfaces. Conversely, upon application of current in a second polarity opposite to the first polarity, the opposite faces of the piezoelectric crystal of the transducer respond by contracting, or drawing, inwards to define respective concave surfaces. In the context of an aerosol-generating apparatus, driving the piezoelectric transducer in the first polarity may ensure that physical contact between the transducer and the received aerosol precursor can be maintained. Maintaining this physical contact may improve the power efficiency of the inducement of cavitation in the aerosol precursor, and therefore may improve the efficiency of the generation of the aerosol. To this end, the driving signal may be a direct current signal to ensure driving of the piezoelectric transducer is carried out in a single polarity.

In the example where the driving signal is a direct current signal, the timing at which the direct current signal is turned on and off, i.e., the driving duty cycle, will affect the frequency components present in the driving signal. For example, a duty cycle of 50% may include frequency components that would not be present for a duty cycle of 40%. The reduction of such additional frequency components may prevent, or minimise, destructive interference between the driving frequency and the additional frequency components, thereby leading to an increase in driving efficiency of the piezoelectric transducer.

In particular, the response of the piezoelectric transducer to the driving signal may include partial oscillations of the piezoelectric transducer. If the driving signal is cut off, due to the duty cycle, partway through one of these partial oscillations, the resulting frequency components from the partial oscillations may interfere with the driving frequency and reduce the efficiency of the piezoelectric transducer. Therefore, the duty cycle may be adjusted, or tuned, to turn off the driving signal at a node of one of the oscillations, minimizing or eliminating the additional frequency components.

By storing the driving duty cycle, and in particular an optimal driving duty cycle, locally to the piezoelectric transducer it may be ensured that the piezoelectric transducer is driven at the optimal driving duty cycle, such as the duty cycle that minimizes or eliminates the additional frequency components in the response of the piezoelectric transducer to the driving signal, rather than having to identify the optimal driving duty cycle of the piezoelectric transducer each time it is driven or provided to a new aerosol generating device.

The driving frequency and the driving duty cycle are interdependent driving parameters for driving the piezoelectric transducer. Put another way, different driving frequencies may have different optimal driving duty cycles. Accordingly, a change in the driving frequency may require a corresponding change in the driving duty cycle in order to produce the target response of the piezoelectric transducer to the driving signal.

By storing both the driving frequency and the driving duty cycle locally to the piezoelectric transducer it may be ensured that the piezoelectric transducer is driven at the optimal combination of driving frequency and driving duty cycle, rather than having to identify the optimal combination of driving frequency and driving duty cycle of the piezoelectric transducer each time it is driven or provided to a new aerosol generating device.

In some examples, the set of one or more driving parameters further comprises a driving power. The driving power at which the driving signal drives the piezoelectric transducer affects one or more properties of the aerosol generated by the aerosol generating apparatus- e.g., according to a user's preference, manufacturer/provider's recommendation, and/or regulatory requirement. Adjusting the driving power may be particularly suitable for adjusting an amount of aerosol generated by a piezoelectric transducer.

By storing the driving power, and in particular an optimal driving power, locally to the piezoelectric transducer it may be ensured that the piezoelectric transducer is driven at the optimal driving power, such as the power that minimizes or eliminates the additional frequency components in the response of the piezoelectric transducer to the driving signal, rather than having to identify the optimal driving power of the piezoelectric transducer each time it is driven or provided to a new aerosol generating device.

The driving frequency, the driving duty cycle and the driving power are all interdependent driving parameters for driving the piezoelectric transducer. Put another way, different driving frequencies may have different optimal driving duty cycles and different optimal driving powers. Accordingly, a change in the driving frequency may require a corresponding change in the driving duty cycle and/or the driving power in order to produce the target response of the piezoelectric transducer to the driving signal.

By storing the driving frequency, the driving duty cycle and the driving power locally to the piezoelectric transducer it may be ensured that the piezoelectric transducer is driven at the optimal combination of driving frequency, driving duty cycle and driving power, rather than having to identify the optimal combination of driving frequency, driving duty cycle and driving power of the piezoelectric transducer each time it is driven or provided to a new aerosol generating device.

In some examples, the tank comprises a first container for containing a first liquid aerosol precursor and a second container for containing a second liquid aerosol precursor. In this case, the aerosol generating unit may comprise a first aerosol generator for generating a first aerosol from the first liquid aerosol precursor; and a second aerosol generator for generating a second aerosol from the second liquid aerosol precursor. In some examples, the first liquid aerosol precursor comprises a nicotine formulation. In some examples, the second liquid aerosol precursor comprises a flavour formulation.

In this way, the device may produce two different aerosols, such as a first aerosol containing nicotine and a second aerosol containing flavour, and not containing nicotine, in order to form a mixed aerosol for inhalation by the user. Generating multiple aerosols independently of each other allows the formation of each individual aerosol to be controlled with greater accuracy.

In some examples, the first aerosol generator comprises a first piezoelectric transducer and the second aerosol generator comprises a second piezoelectric transducer. In some examples, the set of one or more driving parameters comprises a first subset of one or more driving parameters for driving the first aerosol generator and a second subset of one or more driving parameters for driving the second aerosol generator.

As outlined above, the optimal driving parameters, such as the optimal driving frequency (i.e., the resonant frequency of a piezoelectric transducer) may shift or change over the lifetime of a piezoelectric transducer. For an aerosol generating device comprising more than one piezoelectric transducer, the optimal driving parameters for each piezoelectric transducer may shift or change differently over time, due to variations and tolerances in the manufacturing process. Accordingly, by maintaining an independent sub-set of driving parameters for each of the different piezoelectric transducers of the aerosol generating device in the cartridge memory unit, it may be ensured that each piezoelectric transducer is driven as efficiently as possible, thereby improving the efficiency of the aerosol generating device as a whole.

In some examples, the first subset of one or more driving parameters comprises a first driving frequency, and wherein the second subset of one or more driving parameters comprises a second driving frequency, and wherein the first driving frequency is different to the second driving frequency. In some examples, the first driving frequency is higher than the second driving frequency.

In this way, the driving frequencies used for generating the first and second aerosols may be different from each other. In the case where the first liquid aerosol precursor comprises a nicotine formulation and the second liquid aerosol precursor comprises a flavour formulation, the first aerosol may be generated with a higher frequency than the second aerosol, such that the first aerosol has a smaller particle size than the second aerosol. In this way for example, the nicotine containing aerosol may comprise particle sizes suitable for pulmonary penetration, i.e., delivery to the lungs, and the nicotine-free aerosol, i.e., the flavour aerosol, may comprises particle sizes suitable for oral deposition.

In some examples, the replaceable cartridge may form part of an aerosol generating system. In this case, the aerosol generating system may comprise an aerosol generating device adapted to receive the replaceable cartridge, the aerosol generating device comprising: a drive circuit for driving the aerosol generating unit; a device communication unit adapted to communicate with the cartridge communication unit to receive the set of one or more driving parameters; and electrical circuitry adapted to: generate a control signal for causing the drive circuit to drive the aerosol generating unit according to the set of one or more driving parameters received from the replaceable cartridge.

In this way, the device may receive the driving parameters stored on the replaceable cartridge for generating the driving signal for driving the aerosol generating unit.

In some examples, the electrical circuitry is further adapted to: vary each of the driving parameters; measure a response of the piezoelectric transducer to the variation of each of the parameters; and determine a set of optimal driving parameters based on the response of the piezoelectric transducer to the variation of each of the parameters.

For example, determining the optimal driving frequency may involve identifying one of the resonant frequencies of the piezoelectric transducer by identifying a maximum amplitude in the piezoelectric transducer's response. Identifying the maximum amplitude in the response of the piezoelectric transducer to the driving signal may involve determining a maximum amplitude in the voltage response of the piezoelectric transducer (i.e., the maximum voltage drop across the piezoelectric transducer). Alternatively, identifying the maximum amplitude may involve determining a maximum amplitude in the current response of the piezoelectric transducer (i.e., the maximum current flow through the piezoelectric transducer).

Alternatively, identifying one of the resonant frequencies of the piezoelectric transducer may involve identifying a minimum amplitude in the impedance response of the piezoelectric transducer (i.e., the minimum impedance - as a function of driving frequency - of the piezoelectric transducer). The minimum amplitude in the impedance response of the piezoelectric transducer may be identified by measuring the impedance response of a component connected in series with the piezoelectric transducer in order to minimize the change in behaviour of the piezoelectric transducer as a result of connecting electrical components to the piezoelectric transducer.

Different piezoelectric transducers may have different dimensions and/or be formed from materials having different compositions and/or structures. As such, different piezoelectric transducers may have different resonant frequencies. Further, over time of use, the resonant frequency/frequencies of a piezoelectric transducer may shift as discussed above, for example, as the material degrades or erodes.

As such, it may be beneficial to identify a piezoelectric transducer's resonant frequency (e.g., the fundamental resonant frequency) as the optimal driving frequency, and to track the piezoelectric transducer's resonant frequency over time, to ensure that the piezoelectric transducer is driven, and continues to be driven, with a driving frequency that ensures an efficient generation of aerosol.

For example, determining the optimal driving duty cycle may involve identifying a duty cycle that produces a maximum amplitude in the piezoelectric transducer's response. Identifying the maximum amplitude in the response of the piezoelectric transducer to the driving signal may involve determining a maximum amplitude in the voltage response of the piezoelectric transducer (i.e., the maximum voltage drop across the piezoelectric transducer). Alternatively, identifying the maximum amplitude may involve determining a maximum amplitude in the current response of the piezoelectric transducer (i.e., the maximum current flow through the piezoelectric transducer).

For example, determining the optimal combination of driving frequency and driving duty cycle may comprises the steps for determining the optimal driving frequency, i.e., the resonant frequency, as outlined above, followed by the steps for determining the optimal duty cycle for the determined optimal driving frequency, as outlined above.

For example, determining the optimal combination of driving frequency and driving duty cycle may comprises the steps for determining the optimal driving frequency, i.e., the resonant frequency, as outlined above, followed by the steps for determining the optimal duty cycle for the determined optimal driving frequency, as outlined above.

The aerosol generating device may then determine the optimal driving power for the determined optimal driving frequency and determined optimal driving duty cycle. Determining the optimal driving power may involve identifying a driving power that produces a maximum amplitude in the piezoelectric transducer's response. Identifying the maximum amplitude in the response of the piezoelectric transducer to the driving signal may involve determining a maximum amplitude in the voltage response of the piezoelectric transducer (i.e., the maximum voltage drop across the piezoelectric transducer). Alternatively, identifying the maximum amplitude may involve determining a maximum amplitude in the current response of the piezoelectric transducer (i.e., the maximum current flow through the piezoelectric transducer).

In some examples, the device communication unit is adapted to communicate the set of optimal driving parameters to the cartridge communication unit, and wherein the cartridge memory unit is adapted to replace the set of one or more driving parameters with the optimal driving parameters.

In this way, the device may be adapted to perform the computationally intensive process of determining an optimal set of driving parameters for a given piezoelectric transducer, which may then be stored on the replaceable cartridge comprising said piezoelectric transducer.

In some examples, the device communication unit communicates wirelessly with the cartridge communication unit. In this way, the driving parameters may be transferred between the aerosol generating device and the replaceable cartridge without requiring a separate wireless connection. For example, the device communication unit may communicate with the cartridge communication unit by way of a near field communication (NFC) protocol, which may also be used to power the cartridge memory unit.

The present disclosure may provide a method driving a piezoelectric transducer of a replaceable cartridge of an aerosol generating device, which may implement any one or more features disclosed herein. The method may comprise communicating a set of one or more driving parameters to the aerosol generating device for driving the aerosol generating unit from a cartridge memory unit of the replaceable cartridge. The method may further comprise generating a control signal for causing the drive circuit of the aerosol generating device to drive the aerosol generating unit according to the set of one or more driving parameters received from the replaceable cartridge.

The present disclosure may provide electrical circuitry and/or a computer program configured to cause an aerosol generating apparatus/system to perform any method or method step disclosed herein. A computer readable medium comprising the computer program is also disclosed.

In a second aspect the present disclosure provides replaceable cartridge for an aerosol generating device that comprises an aerosol generating unit for generating an aerosol from the liquid aerosol precursor, wherein the aerosol generating unit comprises a piezoelectric transducer.

In the second aspect, the replaceable cartridge comprises a cartridge memory unit adapted to store an inhalation metric associated with the aerosol generating unit.

In the second aspect, the replaceable cartridge comprises a cartridge communication unit adapted to receive an inhalation metric update from the aerosol generating device for updating the inhalation metric stored in the cartridge memory unit.

In other words, there is provided a replaceable cartridge with a memory unit for receiving and storing a metric relating to the inhalations performed by a user of the aerosol generating device.

Put another way, a metric of the inhalations a user has performed with a given replaceable cartridge may be stored locally within a memory on the cartridge itself.

By providing an on-cartridge memory unit the invention provides a means of storing the inhalation metrics in the same, removable, part of the system as the components that are directly affected, or depleted, by user inhalations (such as the aerosol generating unit). In this way, should the replaceable cartridge be separated from a given aerosol generating device and inserted into a different aerosol generating device, the metrics associated with inhalations performed using the given replaceable pod can still be utilized by the aerosol generating device to monitor an aspect of the replaceable cartridge accurately.

In some examples, the tank comprises a first container for containing a first liquid aerosol precursor and a second container for containing a second liquid aerosol precursor. In this case, the aerosol generating unit may comprise a first aerosol generator for generating a first aerosol from the first liquid aerosol precursor; and a second aerosol generator for generating a second aerosol from the second liquid aerosol precursor. In some examples, the first liquid aerosol precursor comprises a nicotine formulation, and the second liquid aerosol precursor comprises a flavour formulation.

In this way, the device may produce two different aerosols, such as a first aerosol containing nicotine and a second aerosol containing flavour, and not containing nicotine, in order to form a mixed aerosol for inhalation by the user. Generating multiple aerosols independently of each other allows the formation of each individual aerosol to be controlled with greater accuracy.

In some examples, the inhalation metric comprises a first inhalation metric associated with the first aerosol generator and a second inhalation metric associated with the second aerosol generator. In this way, the device may accurately track the inhalations metrics for each aerosol generator individually. For example, both aerosol generators may not be driven in the same manner with every inhalation, which may then be more accurately reflected in the separate inhalation metrics.

In some examples, the first aerosol generator comprises a first piezoelectric transducer and the second aerosol generator comprises a second piezoelectric transducer. By using piezoelectric transducers as the first and second aerosol generators, the speed at, or reactivity with, which the first and second aerosol generator can be activated and deactivated may be improved, for example when compared to heaters which have lag times associated with heating up and cooling down.

In some examples, the cartridge memory unit is further adapted to store a driving metric associated with the piezoelectric transducer, and wherein the cartridge communication unit is further adapted to receive a driving metric update from the aerosol generating device for updating the driving metric stored in the cartridge memory unit.

Piezoelectric transducers require a combination of interrelated driving parameters in order to be driven at an optimal efficiency. Each of the driving parameters has an effect on each of the other driving parameters, meaning that tuning a driving signal for driving a piezoelectric transducer in an optimal manner is a complex task as an adjustment to any of the driving parameters will have a knock-on effect on the remaining driving parameters.

In the context of an aerosol generating device, producing a target response in the piezoelectric transducer may result in a target aerosol generation parameter being met, for example, a target aerosol volume or a target aerosol droplet size within the generated aerosol.

However, during the lifetime of a piezoelectric transducer, the resonant frequency of said piezoelectric transducer may shift over time. Such a shift in resonant frequency may occur on a short-term scale, for example due to the piezoelectric transducer heating up during a single usage session, and may also occur on a long-term scale, for example where the resting resonance frequency (i.e., the resonance frequency of the piezoelectric transducerwhen it is not in use) changes over time due to material fatigue.

By providing an on-cartridge memory unit the invention provides a means of storing the driving metrics in the same, removable, part of the system as the components that are directly affected, or depleted, by user inhalations (such as the piezoelectric transducer). In this way, should the replaceable cartridge be separated from a given aerosol generating device and inserted into a different aerosol generating device, the metrics associated with the usage of the piezoelectric transducer can still be utilized by the aerosol generating device to monitor the lifetime usage of the piezoelectric transducer accurately.

In some examples, the replaceable cartridge may form part of an aerosol generating system. In this case, the aerosol generating system may comprise an aerosol generating device adapted to receive the replaceable cartridge, the aerosol generating device comprising: a sensor for detecting an inhalation of the user; a drive circuit for driving the aerosol generating unit according to a set of one or more driving parameters in response to a detected inhalation; a electrical circuitry adapted to: generate an inhalation count based on a number of inhalations detected by the sensor; monitor the driving of the aerosol generating unit to determine an aerosol generation efficiency for each inhalation; and generate an inhalation metric update based on the inhalation count and the aerosol generation efficiency; and a device communication unit adapted to communicate with the cartridge communication unit for communicating the inhalation metric update to the cartridge communication unit.

By generating the inhalation metric as a combination of inhalation count and aerosol generation efficiency, the device may more accurately determine the consumption of aerosol and aerosol precursor liquid by the user, as opposed to a plain inhalation count. Accordingly, the device may more accurately derive information about the user's consumption as well as more accurately track the remaining amount of liquid aerosol precursor in the replaceable cartridge.

In some examples, monitoring the driving of the aerosol generating unit comprises measuring a response of the piezoelectric transducer to the set of one or more driving parameters for driving the piezoelectric transducer. The set of one or more driving parameters may comprise one or more of: a driving frequency; a driving duty cycle; and a driving power.

The driving frequency at which the piezoelectric transducer is driven affects one or more properties of the aerosol generated by the aerosol generating apparatus - e.g., according to a user's preference, manufacturer/provider's recommendation, and/or regulatory requirement. Adjusting the driving frequency may be particularly suitable for adjusting an average size of droplets of the aerosol precursor entrained in the generated aerosol and/or a distribution of the size of said droplets. The inventors have observed that the frequency of the driving signal is a parameter that directly affects the average size of droplets in the aerosol generated by the aerosol generating apparatuses described herein. In particular, when driving the piezoelectric transducer with a driving signal having a relatively higher driving frequency, a relatively smaller average droplet size is observed. This observation is consistent with the physical mechanism for cavitation described in Kooij et al. Sci. Rep., 9, 6128 (2019), the entirety of which is incorporated herein by reference.

For example, measuring the response of the piezoelectric transducer to the driving frequency may include measuring an amplitude in the piezoelectric transducer's response. Measuring the amplitude may involve measuring an amplitude in the voltage response of the piezoelectric transducer (i.e., the maximum voltage drop across the piezoelectric transducer). Alternatively, measuring an amplitude may involve measuring an amplitude in the current response of the piezoelectric transducer (i.e., the maximum current flow through the piezoelectric transducer).

Alternatively, measuring the response of the piezoelectric transducer may involve measuring an impedance response of the piezoelectric transducer (i.e., the minimum impedance - as a function of driving frequency - of the piezoelectric transducer).

The driving duty cycle at which the driving signal drives the piezoelectric transducer affects one or more properties of the aerosol generated by the aerosol generating apparatus - e.g., according to a user's preference, manufacturer/provider's recommendation, and/or regulatory requirement. Adjusting the driving duty cycle may be particularly suitable for adjusting an amount of aerosol generated by a piezoelectric transducer.

For example, measuring the response of the piezoelectric transducer to the driving duty cycle may include measuring an amplitude in the piezoelectric transducer's response. Measuring the amplitude may involve measuring an amplitude in the voltage response of the piezoelectric transducer (i.e., the maximum voltage drop across the piezoelectric transducer). Alternatively, measuring an amplitude may involve measuring an amplitude in the current response of the piezoelectric transducer (i.e., the maximum current flow through the piezoelectric transducer).

The driving power at which the driving signal drives the piezoelectric transducer affects one or more properties of the aerosol generated by the aerosol generating apparatus- e.g., according to a user's preference, manufacturer/provider's recommendation, and/or regulatory requirement. Adjusting the driving power may be particularly suitable for adjusting an amount of aerosol generated by a piezoelectric transducer.

For example, measuring the response of the piezoelectric transducer to the driving power may include measuring an amplitude in the piezoelectric transducer's response. Measuring the amplitude may involve measuring an amplitude in the voltage response of the piezoelectric transducer (i.e., the maximum voltage drop across the piezoelectric transducer). Alternatively, measuring an amplitude may involve measuring an amplitude in the current response of the piezoelectric transducer (i.e., the maximum current flow through the piezoelectric transducer).

In some examples, generating the inhalation metric update comprises: comparing the response of the piezoelectric transducer to the set of one or more driving parameters to an expected response of the piezoelectric transducer to the set of one or more driving parameters; and generating the aerosol generation efficiency based on the comparison between the response of the piezoelectric transducer and the expected response of the piezoelectric transducer.

For example, an expected response of the piezoelectric transducer to a given set of driving parameters may be stored in a look-up table in a memory of the aerosol generating device. The aerosol generation efficiency may be calculated as a percentage or ratio between the measured response of the piezoelectric transducer to the set of one or more driving parameters to the expected response of the piezoelectric transducer to said driving parameters.

In this way, a more accurate indication of how much aerosol, and aerosol precursor liquid, consumed may be generated. For example, if a replaceable cartridge were to be assumed to contain 100 inhalations worth of aerosol precursor liquid, an aerosol generating device monitoring only inhalation count may assume that the replaceable cartridge is empty after 100 inhalations. However, if each inhalation were to only have an aerosol generation efficiency of 50%, then in fact only half of the aerosol precursor liquid would have been consumed after 100 inhalations. Accordingly, by accounting for the aerosol generation efficiency, the monitoring of aerosol, and aerosol precursor liquid, consumption may be made more accurate.

In some examples, the cartridge communication unit is further adapted to communicate an updated inhalation metric to the aerosol generating device, and wherein the electrical circuitry is further adapted to generate a control signal for adjusting a behaviour of the aerosol generating device. Accordingly, the device may be adapted to control the generation of aerosol based on the monitored inhalation metric, which may be indicative of the amount of aerosol, and aerosol precursor liquid, consumed.

In some examples, the electrical circuitry is further adapted to: compare the updated inhalation metric to an inhalation metric threshold; and if the updated inhalation metric is greater than or equal to the inhalation metric threshold, determine that the replaceable cartridge is depleted; and if the updated inhalation metric is less than the inhalation metric threshold, determine that the replaceable cartridge is not depleted. In some examples, in response to determining that the replaceable cartridge is depleted, the electrical circuitry is further adapted to prevent the driving circuit from driving the piezoelectric transducer.

In this way, the device may more accurately generate an indication of when the replaceable cartridge is empty of aerosol precursor liquid and prevent further attempted use of the aerosol generating device until the replaceable cartridge is replaced.

In some examples, the electrical circuitry is further adapted to: generate a drive count based on a number of driving signals sent to the piezoelectric transducer; monitor the driving of the aerosol generating unit to determine a driving period for each driving signal; and generate the drive metric update based on the drive count and the aerosol driving period.

In this way, a more accurate indication of the fatigue of the piezoelectric transducer over time may be generated. For example, if a piezoelectric transducer were to be assumed to be exhausted after 100 two second inhalations, an aerosol generating device monitoring only inhalation count may assume that the piezoelectric transducer is empty after 100 inhalations of any length. However, if each inhalation were to only be one second in duration, then in fact the piezoelectric transducer would not be exhausted and could continue to be used. Alternatively, if each inhalation were to be four seconds in duration, then in fact the piezoelectric transducer would be exhausted after 50 inhalations. Accordingly, by accounting for the driving period, the monitoring of the state of the piezoelectric transducer may be made more accurate.

In some examples, the cartridge communication unit is further adapted to communicate an updated drive metric to the aerosol generating device, and wherein the electrical circuitry is further adapted to: compare the updated drive metric to a drive metric threshold; and if the updated drive metric is greater than or equal to the drive metric threshold, determine that the piezoelectric transducer is exhausted; and if the updated drive metric is less than the drive metric threshold, determine that the piezoelectric transducer is not exhausted.

In this way, the device may more accurately generate an indication of when the piezoelectric transducer is exhausted and prevent further attempted use of the aerosol generating device until the piezoelectric transducer, or replaceable cartridge, is replaced.

The present disclosure may provide a method of generating an inhalation metric update for an aerosol generating system, which may implement any one or more features disclosed herein. The method may comprise generating an inhalation count based on a number of inhalations detected by the sensor; monitoring the driving of the aerosol generating unit to determine an aerosol generation efficiency for each inhalation; and generating an inhalation metric update based on the inhalation count and the aerosol generation efficiency.

The present disclosure may provide electrical circuitry and/or a computer program configured to cause an aerosol generating apparatus/system to perform any method or method step disclosed herein. A computer readable medium comprising the computer program is also disclosed.

In embodiments, the electrical circuitry is implemented as one or more processors, which are configured to implement the disclosed steps, e.g. as the controller. The processors may execute program code stored on electronic memory and/or may execute logic, e.g. as a logic array, gate array, structured gate array.

The preceding summary is provided for purposes of summarizing some examples to provide a basic understanding of aspects of the subject matter described herein. Accordingly, the above-described features should not be construed to narrow the scope or spirit of the subject matter described herein in any way. Moreover, the above and/or proceeding examples may be combined in any suitable combination to provide further examples, except where such a combination is clearly impermissible or expressly avoided. Other features, aspects, and advantages of the subject matter described herein will become apparent from the following text and the accompanying drawings.

### BRIEF DESCRIPTION OF THE FIGURES

Aspects, features and advantages of the present disclosure will become apparent from the following description of examples in reference to the appended drawings in which like numerals denote like elements.
Fig. 1 is a block system diagram showing an example aerosol generating apparatus.
Fig. 2 is a block system diagram showing an example implementation of the apparatus of Fig. 1, where the aerosol generating apparatus is configured to generate aerosol from a liquid precursor.
Figs. 3A and 3B are schematic diagrams showing an example implementation of the apparatus of Fig. 2.
Fig. 4 is a block system diagram showing an example system for managing an aerosol generating apparatus.
Fig. 5 shows an example of a circuit for modelling the behaviour of an exemplary piezoelectric transducer.
Fig. 6 shows a portion of an exemplary driving circuit using an H-bridge.
Fig. 7 shows an example of an improved driving circuit for driving a piezoelectric transducer.
Fig. 8 shows an example of an aerosol generating system.
Fig. 9 shows an example of a method for controlling the aerosol generating system of Fig. 8.
Fig. 10 shows an example of a method for controlling the aerosol generating system of Fig. 8.
Fig. 11 shows a further example of a method for controlling the aerosol generating system of Fig. 8.

### DETAILED DESCRIPTION OF EMBODIMENTS

Before describing several examples implementing the present disclosure, it is to be understood that the present disclosure is not limited by specific construction details or process steps set forth in the following description and accompanying drawings. Rather, it will be apparent to those skilled in the art having the benefit of the present disclosure that the systems, apparatuses and/or methods described herein could be embodied differently and/or be practiced or carried out in various alternative ways.

Unless otherwise defined herein, scientific and technical terms used in connection with the presently disclosed inventive concept(s) shall have the meanings that are commonly understood by those of ordinary skill in the art, and known techniques and procedures may be performed according to conventional methods well known in the art and as described in various general and more specific references that may be cited and discussed in the present specification.

Any patents, published patent applications, and non-patent publications mentioned in the specification are hereby incorporated by reference in their entirety.

All examples implementing the present disclosure can be made and executed without undue experimentation in light of the present disclosure. While particular examples have been described, it will be apparent to those of skill in the art that variations may be applied to the systems, apparatus, and/or methods and in the steps or in the sequence of steps of the methods described herein without departing from the concept, spirit, and scope of the inventive concept(s). All such similar substitutions and modifications apparent to those skilled in the art are deemed to be within the spirit, scope, and concept of the inventive concept(s) as defined by the appended claims.

The use of the term "a" or "an" in the claims and/or the specification may mean "one," as well as "one or more," "at least one," and "one or more than one." As such, the terms "a," "an," and "the," as well as all singular terms, include plural referents unless the context clearly indicates otherwise. Likewise, plural terms shall include the singular unless otherwise required by context.

The use of the term "or" in the present disclosure (including the claims) is used to mean an inclusive "and/or" unless explicitly indicated to refer to alternatives only or unless the alternatives are mutually exclusive. For example, a condition "A or B" is satisfied by any of the following: A is true (or present) and B is false (or not present), A is false (or not present) and B is true (or present), and both A and B are true (or present).

As used in this specification and claim(s), the words "comprising, "having," "including," or "containing" (and any forms thereof, such as "comprise" and "comprises," "have" and "has," "includes" and "include," or "contains" and "contain," respectively) are inclusive or open-ended and do not exclude additional, unrecited elements or method steps.

Unless otherwise explicitly stated as incompatible, or the physics or otherwise of the embodiments, examples, or claims prevent such a combination, the features of examples disclosed herein, and of the claims, may be integrated together in any suitable arrangement, especially ones where there is a beneficial effect in doing so. This is not limited to only any specified benefit, and instead may arise from an "ex post facto" benefit. This is to say that the combination of features is not limited by the described forms, particularly the form (e.g. numbering) of example(s), embodiment(s), or dependency of claim(s). Moreover, this also applies to the phrase "in one embodiment," "according to an embodiment," and the like, which are merely a stylistic form of wording and are not to be construed as limiting the following features to a separate embodiment to all other instances of the same or similar wording. This is to say, a reference to 'an,' 'one,' or 'some' embodiment(s) may be a reference to any one or more, and/or all embodiments, or combination(s) thereof, disclosed. Also, similarly, the reference to "the" embodiment may not be limited to the immediately preceding embodiment. Further, all references to one or more embodiments or examples are to be construed as non-limiting to the claims.

The present disclosure may be better understood in view of the following explanations, wherein the terms used that are separated by "or" may be used interchangeably:

As used herein, an "aerosol generating apparatus" (or "electronic(e)-cigarette") may be an apparatus configured to deliver an aerosol to a user for inhalation by the user. The apparatus may additionally/alternatively be referred to as a "smoking substitute apparatus", if it is intended to be used instead of a conventional combustible smoking article. As used herein a combustible "smoking article" may refer to a cigarette, cigar, pipe or other article, that produces smoke (an aerosol comprising solid particulates and gas) via heating above the thermal decomposition temperature (typically by combustion and/or pyrolysis). An aerosol generated by the apparatus may comprise an aerosol with particle sizes of 0.2 - 7 microns, such as between 2-3 microns, or less than 10 microns, or less than 7 microns, or less than 3 microns, or less than 2 microns. This particle size may be achieved by control of one or more of: driving parameters of the ultrasonic generator; flow properties including turbulence and velocity. The generation of aerosol by the aerosol generating apparatus may be controlled by an input device. The input device may be configured to be user-activated, and may for example include or take the form of an actuator (e.g. actuation button) and/or an airflow sensor.

Each occurrence of the aerosol generating apparatus being caused to generate aerosol for a period of time (which may be variable) may be referred to as an "activation" of the aerosol generating apparatus. The aerosol generating apparatus may be arranged to allow an amount of aerosol delivered to a user to be varied per activation (as opposed to delivering a fixed dose of aerosol), e.g. by activating an aerosol generating unit of the apparatus for a variable amount of time, e.g. based on the strength/duration of a draw of a user through a flow path of the apparatus (to replicate an effect of smoking a conventional combustible smoking article).

The aerosol generating apparatus may be portable. As used herein, the term "portable" may refer to the apparatus being for use when held by a user.

As used herein, an "aerosol generating system" may be a system that includes an aerosol generating apparatus and optionally other circuitry/components associated with the function of the apparatus, e.g. one or more external devices and/or one or more external components (here "external" is intended to mean external to the aerosol generating apparatus).

As used herein, an "external device" and "external component" may include one or more of a: a charging device, a mobile device (which may be connected to the aerosol generating apparatus, e.g. via a wireless or wired connection); a networked-based computer (e.g. a remote server); a cloud-based computer; any other server system.

An example aerosol generating system may be a system for managing an aerosol generating apparatus. Such a system may include, for example, a mobile device, a network server, as well as the aerosol generating apparatus.

As used herein, an "aerosol" may include a suspension of liquid droplets of precursor. An aerosol may include one or more components of the precursor.

As used herein, a "precursor" may include one or more of a: liquid; and a gel. The precursor may be processed by an aerosol generating unit of an aerosol generating apparatus to generate an aerosol. The precursor may include one or more of: an active component; a carrier; a flavouring. The active component may include one or more of nicotine; caffeine; a cannabidiol oil; a non-pharmaceutical formulation, e.g. a formulation which is not for treatment of a disease or physiological malfunction of the human body. The active component may be carried by the carrier, which may be a liquid, including propylene glycol and/or glycerine. The term "flavouring" may refer to a component that provides a taste and/or a smell to the user. The flavouring may include one or more of: Ethylvanillin (vanilla); menthol, Isoamyl acetate (banana oil); or other. The precursor may include a carrier; a flavouring.

As used herein, a "storage portion" may be a portion of the apparatus adapted to store the precursor. It may be implemented as fluid-holding reservoir depending on the implementation of the precursor as defined above.

As used herein, a "flow path" may refer to a path or enclosed passageway through an aerosol generating apparatus, e.g. for delivery of an aerosol to a user. The flow path may be arranged to receive aerosol from an aerosol generating unit. When referring to the flow path, upstream and downstream may be defined in respect of a direction of flow in the flow path, e.g. with an outlet being downstream of an inlet.

As used herein, a "delivery system" may be a system operative to deliver an aerosol to a user. The delivery system may include a mouthpiece and a flow path. The delivery system may be at least partly within the aerosol generating component.

As used herein, a "flow" may refer to a flow in a flow path. A flow may include aerosol generated from the precursor. The flow may include air, which may be induced into the flow path via a puff by a user.

As used herein, a "puff" (or "inhale" or "draw") by a user may refer to expansion of lungs and/or oral cavity of a user to create a pressure reduction that induces flow through the flow path.

As used herein, an "aerosol generating unit" may refer to a device configured to generate an aerosol from a precursor. The aerosol generating unit may include a unit to generate an aerosol directly from the precursor (e.g. an atomiser including an ultrasonic system). A plurality of aerosol generating units to generate a plurality of aerosols (for example, from a plurality of different aerosol precursors) may be present in an aerosol generating apparatus.

As used herein, an "ultrasonic generator" may refer to a piezoelectric transducer capable of vibrating at ultrasonic frequencies, i.e., at frequencies greater than 20kHz. In some examples, the piezoelectric transducer may be capable of vibrating at even higher frequencies, e.g., at frequencies of 100 kHz or above, 500 kHz or above, 1 MHz or more, 2 MHz or more, 5 MHz or more, or 10 MHz or more. The piezoelectric transducer may be adapted to vibrate in response to a driving signal, and in particular adapted to vibrate at the frequency of the driving signal. In some examples, the driving signal may be generated by direct digital synthesis (DDS).

As used herein, a "piezoelectric transducer" may refer to an ultrasonic transducer comprising a piezoelectric crystal, which generates a mechanical strain internally in response to an electric field. A rapidly changing electric field, such as an ultrasonic frequency driving signal, results in rapidly changing mechanical strain within the piezoelectric crystal causing it to vibrate. The piezoelectric transducer will have an aerosolisation surface from which the aerosol is generated. The aerosolisation surface typically faces into an aerosolisation chamber.

As used herein, an "**aerosol generating component"** may refer to a component that includes an aerosol precursor. The component may include an aerosol generating unit e.g. it may be arranged as a cartomizer. The component may include a mouthpiece. The component may include an information carrying medium. The component may include a storage portion, e.g. a reservoir or tank, for storage of the aerosol precursor.

With liquid or gel implementations of the aerosol precursor, e.g. an e-liquid, the component may be referred to as a "capsule" or a "pod" or an "e-liquid consumable". In some embodiments, the aerosol precursor component may be affixed to the device body to form the aerosol generating apparatus. In these embodiments, the reservoir/tank may be refillable.

The aerosol generating component e.g. the capsule, pod, or consumable may be for releasable coupling to a device body to form the aerosol generating apparatus.

The device body may comprise a power supply for powering the aerosol generating unit.

As used herein, an "information carrying medium" may include one or more arrangements for storage of information on any suitable medium. Examples include: a computer readable medium; a Radio Frequency Identification (RFID) transponder; codes encoding information, such as optical (e.g. a bar code or QR code) or mechanically read codes (e.g. a configuration of the absence or presents of cutouts to encode a bit, through which pins or a reader may be inserted).

As used herein, "electrical circuitry" may refer to one or more electrical components, examples of which may include: an Application Specific Integrated Circuit (ASIC) or other programable logic; electronic/electrical componentry (which may include combinations of transistors, resistors, capacitors, inductors etc); one or more processors (e.g. the circuitry structure of the processor); a non-transitory memory (e.g. implemented by one or more memory devices), that may store one or more software or firmware programs; a combinational logic circuit; interconnection of the aforesaid. The electrical circuitry may be located entirely at the apparatus (e.g., in the device/main body comprising the power supply), or distributed between the apparatus and/or on one or more external devices in communication with the apparatus, e.g. as part of a system. The electrical circuitry may comprise a controller. The controller may be configured to carry out any of the methods described herein.

As used herein, a "processing resource" (or "processor" or "controller") may refer to one or more units for processing data, examples of which may include an ASIC, microcontroller, FPGA, microprocessor, digital signal processor (DSP) capability, state machine or other suitable component. A processing resource may be configured to execute a computer program, e.g. which may take the form of machine readable instructions, which may be stored on a non-transitory memory and/or programmable logic. The processing resource may have various arrangements corresponding to those discussed for the circuitry, e.g. on-board and/or off board the apparatus as part of the system. As used herein, any machine executable instructions, or computer readable media, may be configured to cause a disclosed method to be carried out, e.g. by an aerosol generating apparatus or system as disclosed herein, and may therefore be used synonymously with the term method.

As used herein, an "external device" (or "peripheral device") may include one or more electronic components external to an aerosol generating apparatus. Those components may be arranged at the same location as the aerosol generating apparatus or remote from the apparatus. An external device may comprise electronic computer devices including: a smartphone; a PDA; a video game controller; a tablet; a laptop; or other like device.

As used herein, a "computer readable medium/media" (or "memory" or "data storage") may include any medium capable of storing a computer program, and may take the form of any conventional non-transitory memory, for example one or more of: random access memory (RAM); a CD; a hard drive; a solid state drive; a memory card; a DVD. The memory may have various arrangements corresponding to those discussed for the circuitry /processor. The present disclosure includes a computer readable medium configured to cause an apparatus or system disclosed herein to perform a method as disclosed herein.

As used herein, a "communication resource" (or "communication interface") may refer to hardware and/or firmware for electronic information/data transfer. The communication resource may be configured for wired communication ("wired communication resources") or wireless communication ("wireless communication resource"). Wireless communication resources may include hardware to transmit and receive signals by radio and may include various protocol implementations e.g. the 802.11 standard described in the Institute of Electronics Engineers (IEEE) and Bluetooth^{™} from the Bluetooth Special Interest Group of Kirkland Wash. Wired communication resources may include; Universal Serial Bus (USB); High-Definition Multimedia Interface (HDMI) or other protocol implementations. The apparatus may include communication resources for wired or wireless communication with an external device.

As used herein, a "network" (or "computer network") may refer to a system for electronic information/data transfer between a plurality of apparatuses/devices. The network may, for example, include one or more networks of any type, which may include: a Public Land Mobile Network (PLMN); a telephone network (e.g. a Public Switched Telephone Network (PSTN) and/or a wireless network); a local area network (LAN); a metropolitan area network (MAN); a wide area network (WAN); an Internet Protocol Multimedia Subsystem (IMS) network; a private network; the Internet; an intranet.

It will be appreciated that any of the disclosed methods (or corresponding apparatuses, programs, data carriers, etc.) may be carried out by either a host or client, depending on the specific implementation (i.e. the disclosed methods/apparatuses are a form of communication(s), and as such, may be carried out from either 'point of view', i.e. in corresponding to each other fashion). Furthermore, it will be understood that the terms "receiving" and "transmitting" encompass "inputting" and "outputting" and are not limited to an RF context of transmitting and receiving electromagnetic (e.g. radio) waves. Therefore, for example, a chip or other device or component for realizing embodiments could generate data for output to another chip, device or component, or have as an input data from another chip, device, or component, and such an output or input could be referred to as "transmit" and "receive" including gerund forms, that is, "transmitting" and "receiving," as well as such "transmitting" and "receiving" within an RF context.

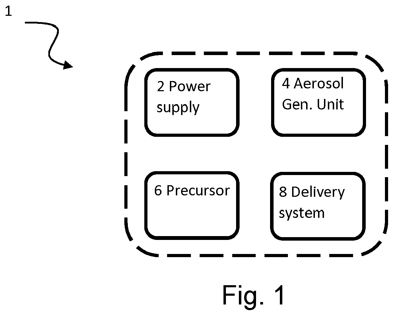

Referring to Fig. 1, an example aerosol generating apparatus 1 includes a power supply 2, for supply of electrical energy. The apparatus 1 includes an aerosol generating unit 4 that is driven by the power supply 2. The power supply 2 may include an electric power supply in the form of a battery and/or an electrical connection to an external power source. The apparatus 1 includes a precursor 6, which in use is aerosolised by the aerosol generating unit 4 to generate an aerosol. The aerosol generating unit 4 includes a piezoelectric transducer (discussed below) configured to induce, by vibration of the piezoelectric transducer i.e. vibration of an aerosolisation surface of the piezoelectric transducer, cavitation in the precursor 6. Collapse of the cavities in the precursor 6 induces a shock that propagates through the liquid precursor 6. This shock disturbs a surface of the liquid precursor 6 that interfaces with air within an aerosolisation chamber of the aerosol generating apparatus 1 (which in turn is in fluid communication with an airflow path within the aerosol generating apparatus). These disturbances take the form of ripples, also known as capillary waves, that form ligaments at the peaks of the ripples/waves, pinch off and expel droplets from the liquid precursor 6 into the airflow path, thereby aerosolising the precursor 6 to generate the aerosol. The apparatus 2 includes a delivery system 8 for delivery of the aerosol to a user.

Electrical circuitry (not shown in figure 1) may be implemented to control the interoperability of the power supply 2 and aerosol generating unit 4.

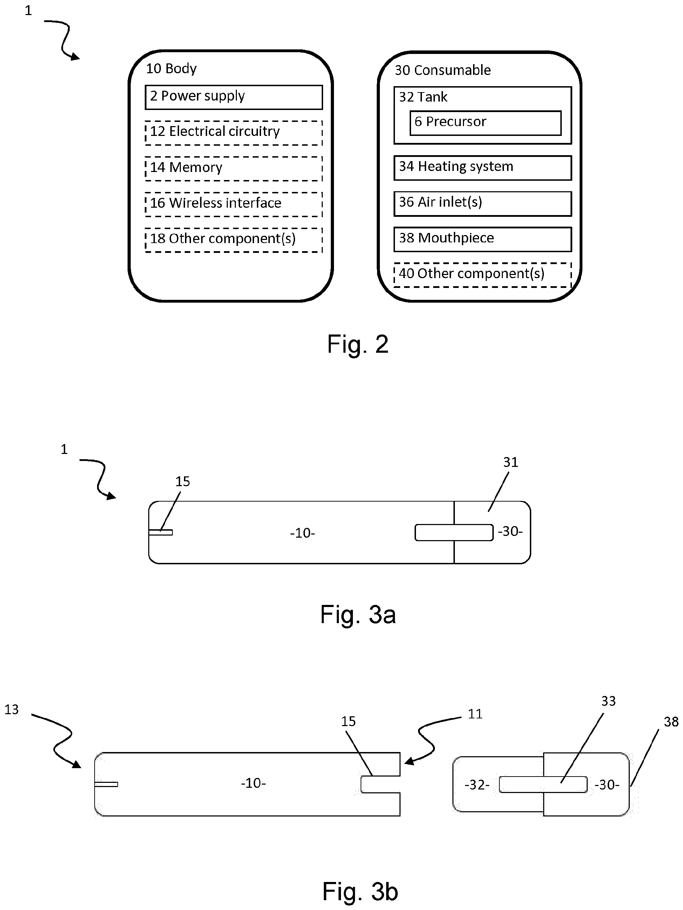

Fig. 2 shows an implementation of the apparatus 1 of Fig. 1, where the aerosol generating apparatus 1 is configured to generate aerosol from a liquid precursor.

In this example, the apparatus 1 includes a device body 10 and a consumable 30.

In this example, the body 10 includes the power supply 2. The body may additionally include any one or more of electrical circuitry 12, a memory 14, a wireless interface 16, one or more other components 18.

The electrical circuitry 12 may include a processing resource for controlling one or more operations of the body 10 and consumable 30, e.g. based on instructions stored in the memory 14.

The wireless interface 16 may be configured to communicate wirelessly with an external (e.g. mobile) device, e.g. via Bluetooth, Bluetooth LE (Low Energy), or WiFi.

The other component(s) 18 may include one or more user interface devices configured to convey information to a user and/or a charging port, for example (see e.g. Fig. 3).

The consumable 30 includes a storage portion implemented here as a tank 32 which stores the liquid precursor 6 (e.g. e-liquid). The consumable 30 also includes one or more air inlets 36, and a mouthpiece 38. The consumable 30 may include one or more other components 40.

The body 10 and consumable 30 may each include a respective electrical interface (not shown) to provide an electrical connection between one or more components of the body 10 with one or more components of the consumable 30. In this way, electrical power can be supplied to components of the consumable 30, without the consumable 30 needing to have its own power supply.

The piezoelectric transducer 34 of the aerosol generating unit 4 is arranged to be in electrical contact with one or more components of the body 10. For example, the power supply 2 may be configured to provide power to the piezoelectric transducer 34. Additionally or alternatively, the piezoelectric transducer 34 may be in electrical contact/communication with one or more of the electrical circuitry 12, memory 14, wireless interface 16 or one or more of the one or more other components 18 e.g., to receive instructions to adjust an operating parameter of the piezoelectric transducer 34 and/or to transmit data indicative of the operational parameters of the piezoelectric transducer 34.

Moreover, the piezoelectric transducer 34 is arranged to be in fluid communication with the tank 32 e.g. via a wick such that the liquid precursor can be provided to the aerosolisation surface of the piezoelectric transducer 34.

In use, a user may activate the aerosol generating apparatus 1 when inhaling through the mouthpiece 38, i.e. when performing a puff. The puff, performed by the user, may initiate a flow through a flow path in the consumable 30 which extends from the air inlet(s) 36 to the mouthpiece 38 via a region (i.e. an aerosolisation chamber) in proximity to the piezoelectric transducer 34.

Activation of the aerosol generating apparatus 1 may be initiated, for example, by an airflow sensor in the body 10 which detects airflow in the aerosol generating apparatus 1 (e.g. caused by a user inhaling through the mouthpiece), or by actuation of an actuator included in the body 10. Upon activation, the electrical circuitry 12 (e.g. under control of the processing resource) may supply electrical energy from the power supply 2 to the piezoelectric transducer 34 of the aerosol generating unit 4, which may cause the piezoelectric transducer 34 to induce cavitation in the liquid precursor 6 drawn from the tank so as to produce an aerosol which is carried by the flow out of the mouthpiece 38.

In some examples, the consumable may include a wick, wherein a first portion of the wick extends into the tank 32 in order to draw liquid precursor 6 out from the tank 32 and wherein a second portion of the wick is arranged to convey the drawn liquid precursor 6 to the aerosolisation surface piezoelectric transducer 34of the aerosol generating unit 4.

In this example, the delivery system 8 is provided by the above-described flow path and mouthpiece 38.

In variant embodiments (not shown), any one or more of the precursor 6, air inlet(s) 36 and mouthpiece 38, may be included in the body 10. For example, the mouthpiece 36 may be included in the body 10 with the precursor 6 arranged as a separable cartomizer.

Figs. 3A and 3B show an example implementation of the aerosol generating apparatus 1 of Fig. 2. In this example, the consumable 30 is implemented as a capsule/pod, which is shown in Fig. 3A as being physically coupled to the body 10, and is shown in Fig. 3B as being decoupled from the body 10.

In this example, the body 10 and the consumable 30 are configured to be physically coupled together by pushing the consumable 30 into an aperture in a top end 11 the body 10, with the consumable 30 being retained in the aperture via an interference fit.

In other examples (not shown), the body 10 and the consumable 30 could be physically coupled together in other ways, e.g. by screwing one onto the other, through a bayonet fitting, or through a snap engagement mechanism, for example.

The body 10 also includes a charging port (not shown) at a bottom end 13 of the body 10.

The body 10 also includes a user interface device configured to convey information to a user. Here, the user interface device is implemented as a light 15, which may e.g. be configured to illuminate when the apparatus 1 is activated. Other user interface devices are possible, e.g. to convey information haptically or audibly to a user.

In this example, the consumable 30 has an opaque cap 31, a translucent tank 32 and a translucent window 33. When the consumable 30 is physically coupled to the body 10 as shown in Fig. 3A, only the cap 31 and window 33 can be seen, with the tank 32 being obscured from view by the body 10. The body 10 includes a slot 15 to accommodate the window 33. The window 33 is configured to allow the amount of liquid precursor 6 in the tank 32 to be visually assessed, even when the consumable 30 is physically coupled to the body 10.

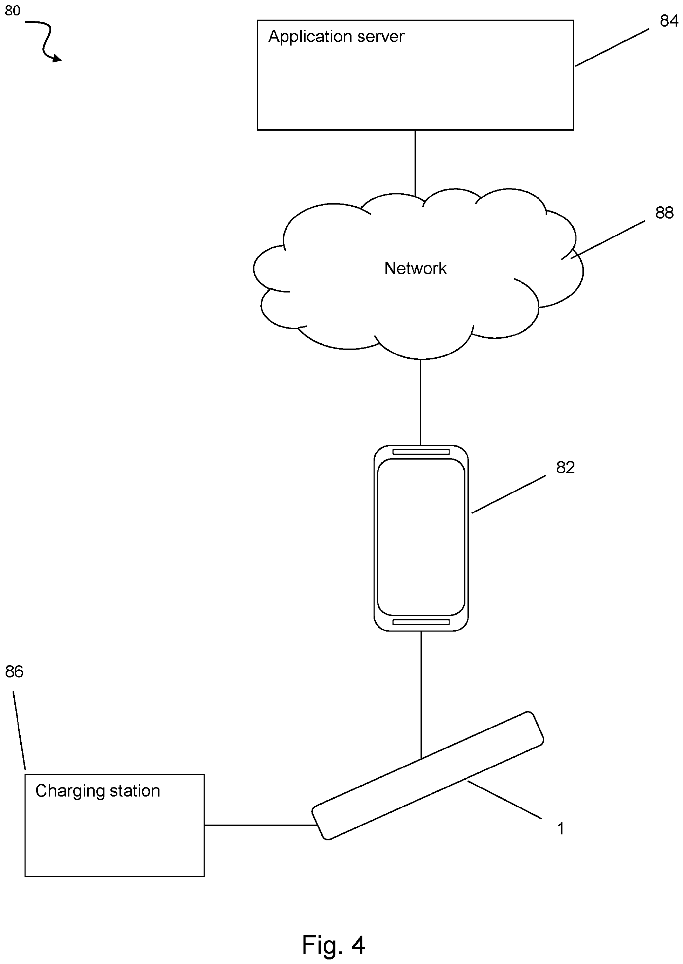

Fig. 4 shows an example system 80 for managing an aerosol generating apparatus 1, such as those described above with reference to any of Figs. 1-3B.

The system 80 as shown in Fig. 1 includes a mobile device 82, an application server 84, an optional charging station 86, as well as the aerosol generating apparatus 1.

In this example, aerosol generating apparatus 1 is configured to communicate wirelessly, e.g. via Bluetooth^{™}, with an application (or "app") installed on the mobile device 2, via a wireless interface included in the aerosol generating apparatus 1 and via a wireless interface included in the mobile device 82. The mobile device 82 may be a mobile phone, for example. The application on the mobile phone is configured to communicate with the application server 84, via a network 88. The application server 84 may utilise cloud storage, for example.

The network 88 may include a cellular network and/or the internet.

In other examples, the aerosol generating apparatus 1 may be configured to communicate with the application server 84 via a connection that does not involve the mobile device 82, e.g. via a narrowband internet of things ("NB-loT") or satellite connection. In some examples, the mobile device 82 may be omitted from the system 80.

A skilled person would readily appreciate that the mobile device 82 may be configured to communicate via the network 88 according to various communication channels, preferably a wireless communication channel such as via a cellular network (e.g. according to a standard protocol, such as 3G or 4G) or via a WiFi network.

The app installed on the mobile device 82 and the application server 84 may be configured to assist a user with managing their aerosol generating apparatus 1, based on information communicated between the aerosol generating apparatus 1 and the app, information communicated directly between the aerosol generating apparatus 1 and the application server 84, and/or information communicated between the app and the application server 84.

The charging station 86 (if present) may be configured to charge (and optionally communicate with) the aerosol generating apparatus 1, via a charging port on the aerosol generating apparatus 1. The charging port on the smoking substitute device 10 may be a USB port, for example, which may allow the aerosol generating apparatus 1 to be charged by any USB-compatible device capable of delivering power to the aerosol generating apparatus 1 via a suitable USB cable (in this case the USB-compatible device would be acting as the charging station 86). Alternatively, the charging station could be a docking station specifically configured to dock with the aerosol generating apparatus 1 and charge the aerosol generating apparatus 1via the charging port on the aerosol generating apparatus 1.

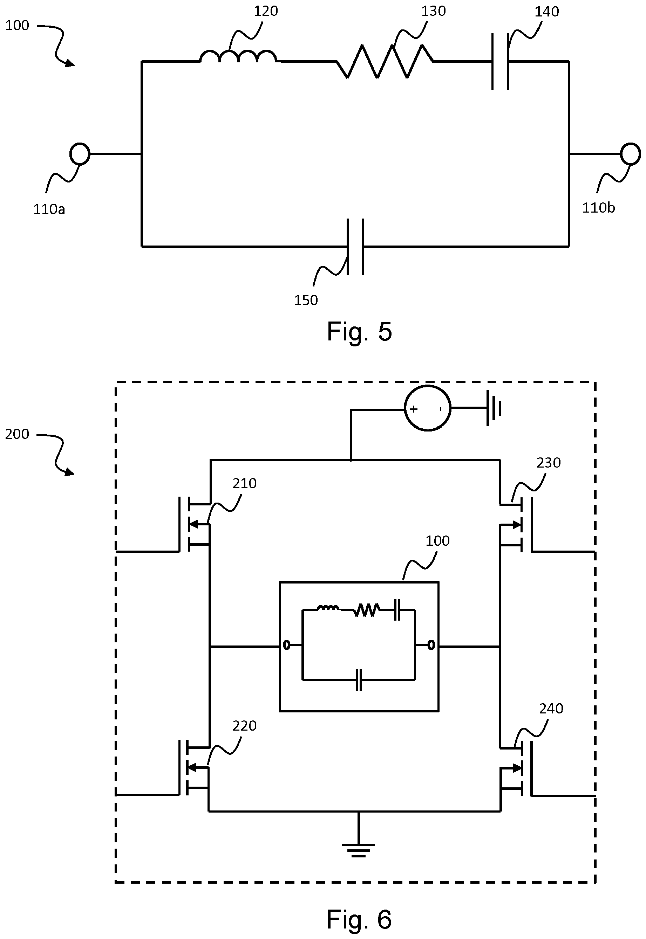

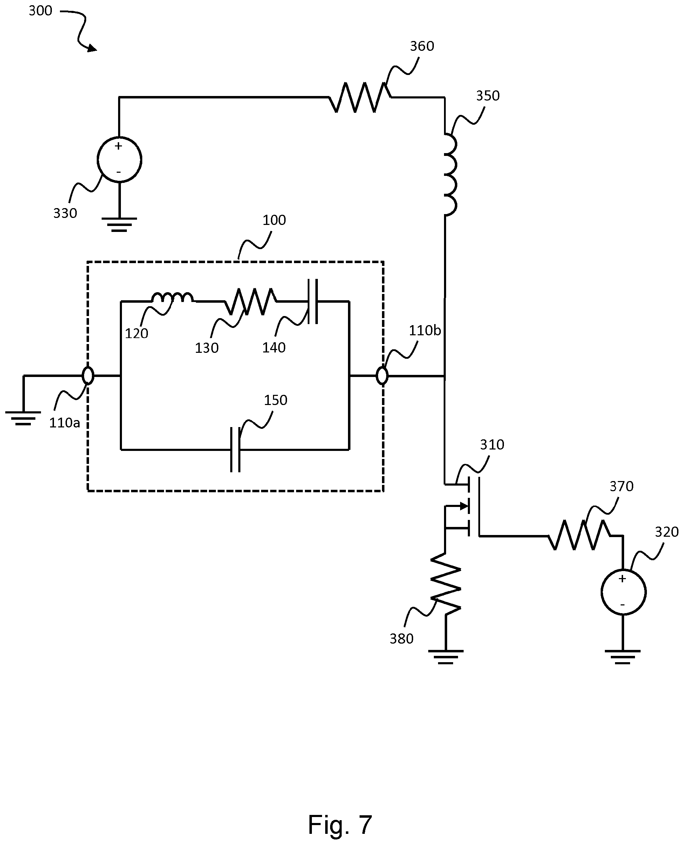

Fig. 5 shows an example of a circuit for modelling the behaviour of a piezoelectric transducer 100 at the resonant frequency of the piezoelectric transducer 100. Example circuits for providing a driving signal to the piezoelectric transducer 100 are described below with reference to Figures 6 and 7.

The circuit includes a set of components connected in series with each other between a pair of terminals 110a, 110b, including: an inductor 120; a resistor 130; and an in-series capacitor 140. The set of series components are connected in parallel with an in-parallel capacitor 150. Each of the components of the circuit model different aspects of the electrical and mechanical behaviour of the piezoelectric transducer 100.

The mechanical vibration of the piezoelectric transducer 100 is modelled by the inductive reactance of the inductor 120, when the frequency of the electric signal driving the piezoelectric transducer 100 is at, or near, the resonant frequency of the piezoelectric transducer 100.

Internal losses associated with the operation of the piezoelectric transducer 100, such as mechanical damping and dielectric losses within the piezoelectric crystal of the transducer 100 are modelled by the resistor 130. The resistance value of the resistor 130 is linked to the quality factor (or Q-factor) of the resonance of the piezoelectric transducer 100, which affects the amplitude and the sharpness of the resonance peak in the transducer's 100 frequency response.

Capacitive mechanical and electrical characteristics of the piezoelectric transducer 100 are modelled by the in-series capacitor 140.

Inherent dielectric properties of the material forming the piezoelectric transducer, e.g., due to the structure of the piezoelectric material between electrodes of the transducer 100 are modelled by the in-parallel capacitor 150. This inherent "parallel" capacitance significantly influences the resonance behaviour of the piezoelectric transducer 100, for example, by affecting the total impedance of the circuit at resonance when combined with the inductive and resistive elements (as modelled by the inductor 120 and the resistor 130).

As an example, the circuit of Figure 5 may be suitable for modelling a typical piezoelectric transducer 100 with a resonance frequency of approximately 3 MHz, by providing the inductor 120 with an inductance of 3 µH, the resistor 130 with a resistance of 4 Ω, the in-series capacitor 140 with a capacitance of 938 pF, and the in-parallel capacitor 150 with a capacitance of 1 nF.

Fig. 6 shows a portion of a conventional driving circuit 200 for driving a piezoelectric transducer 100 using an H-bridge. The H-bridge is defined by four switches 210, 220, 230, 240 arranged in an 'H-shaped' arrangement around the piezoelectric transducer 100. In the example shown in Fig. 6, the four switches 210, 220, 230, 240 are each defined by a respective MOSFET. The H-bridge of Fig. 6 is useful for rapidly changing the polarity of a voltage applied to the piezoelectric transducer 100, thereby driving piezoelectric vibrations in the transducer 100.

H-bridge circuits such as the one depicted in Fig. 6 may face challenges in the context of a user device such as the aerosol-generating apparatus 1 described herein.

For example, high-frequency switching of the four (MOSFET) switches 210, 220, 230, 240 may result in significant power and heat dissipation, generating considerable amounts of heat. This heat can degrade component performance over time and shorten the lifespan of the whole H-bridge, including the piezoelectric transducer 100. Moreover, the power dissipation may represent an undesirable inefficiency in the circuit performance of the H-bridge.

There may also be a risk of a latch-up type short-circuit in which one or more parts of the H-bridge circuit become uncontrollably conductive, thereby compromising the circuit's reliability. In the extreme, latch-up can lead to total circuit failure.

Electromagnetic interference may also be a concern when considering the implementation of a H-bridge. The rapid switching inherent in the operation of the four (MOSFET) switches 210, 220, 230, 240 can generate interference that can disrupt the operation of other electronic components/devices in the vicinity of the H-bridge.

Additionally, the operation of the piezoelectric transducer 100 (or indeed any component having an inductive load), can lead to high-voltage spikes in the current flowing through the circuit. Such spikes risk causing severe damage to the transistors used to embody the four MOSFET switches 210, 220, 230, 240 of the H-bridge shown in Fig. 6.

Furthermore, to induce high-frequency (e.g., ultrasonic) vibrations in the piezoelectric transducer 100, very precise and potentially complex timing control of the H-bridge is required. In particular, if both the first and second switches 210, 220, both the first and third switches 210, 230, both the second and fourth switches 220, 240 or both the third and fourth switches 230, 240 are open at the same time, there is a significant risk of shoot-through, or crossover, current that risks damaging the switches as the shoot-through current passes through and reduces the power efficiency of the H-bridge.

Fig. 7 shows an example of an improved driving circuit 300 for driving a piezoelectric transducer 100 using a single (MOSFET) switch 310.

The driving circuit 300 of Fig. 7 comprises a gate power source 320 configured to controllably apply a voltage to the gate of the MOSFET switch 310 to controllably open and close the MOSFET switch 310. The gate power source 320 may be connected to a clock, or may be an oscillator circuit so as to cyclically open and close the MOSFET switch 310 at a selected frequency.

The driving circuit 300 further comprises a driving power source 330 configured to supply power through the driving circuit 300. When the MOSFET switch 310 is closed, the current supplied by the driving power source 300 bypasses the piezoelectric transducer and flows into the source of the MOSFET switch 310 and out from the drain of the MOSFET switch 310 to ground. The driving power source 330 may be the power supply 2 discussed above in relation to Fig. 1.

When the MOSFET switch 310 is open, the current supplied by the driving power source 300 flows through the piezoelectric transducer 100 to ground, thereby inducing vibration in the piezoelectric transducer.

Application of a current to a piezoelectric transducer 100 induces a mechanical response in the transducer 100. Typically, the current applied to the piezoelectric transducer 100 is an alternating current so as to induce oscillatory vibrations in the piezoelectric transducer 100. Upon application of a current in a first polarity, opposite faces of the piezoelectric crystal of the transducer 100 respond by expanding, or bulging, outwards to define respective convex surfaces. Conversely, upon application of current in a second polarity opposite to the first polarity, the opposite faces of the piezoelectric crystal of the transducer 100 respond by contracting, or drawing, inwards to define respective concave surfaces. In the context of an aerosol-generating apparatus 1, it may be advantageous to only drive the piezoelectric transducer 100 in the first polarity so that physical contact between the transducer 100 and the at least some of the liquid precursor 6 can be maintained. Maintaining this physical contact improves the power efficiency of the inducement of cavitation in the liquid precursor 6, and therefore improves the efficiency of the generation of the aerosol. To this end, the driving signal provided by the driving power source 330 is preferably a direct current power source oscillating, at the piezoelectric transducer 100, between a maximum amplitude and a minimum (zero) amplitude with a frequency corresponding to the switching frequency of the MOSFET switch 310.

The driving circuit 300 may further comprise an inductor 350 connected in series with the piezoelectric transducer. The inductor 350 is arranged and configured with an inductance suitable for smoothing the current profile of the signal provided by the driving power source 330 such that the piezoelectric transducer 100 is not subjected to abrupt step-changes in the voltage and current flowing therethrough. This smoothing of the current profile consequently reduces the risk of damage to the piezoelectric transducer by reducing the risk of harmful voltage spikes.

The driving circuit 300 may further comprise one or more resistors 360, 370, 380 configured to limit the current flowing through the driving circuit.

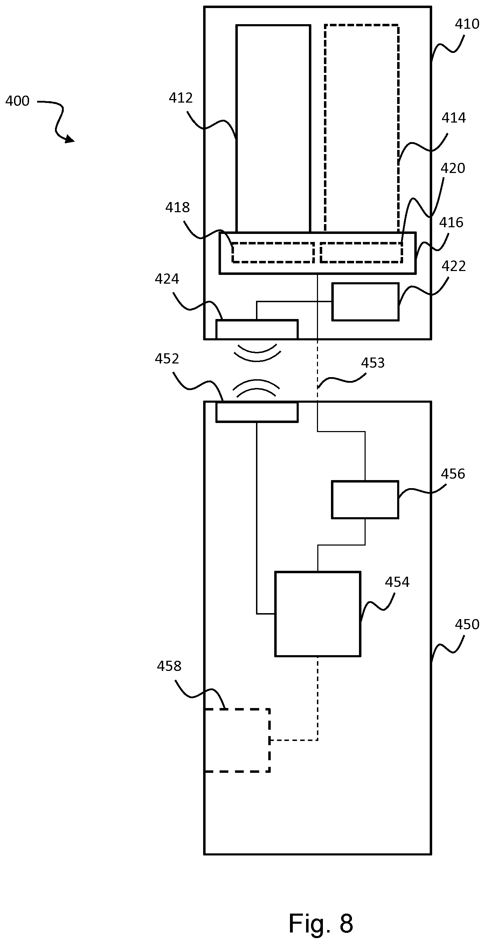

Referring to Fig. 8 there is provided an aerosol generating system 400 that comprises a replaceable cartridge 410 and an aerosol generating device 450 adapted to receive the replaceable cartridge 410.

In the example shown in Fig. 8, the replaceable cartridge 410 comprises a first tank 412 containing a first liquid aerosol precursor and a second tank 414 containing a second liquid aerosol precursor. In the example shown in Fig. 8, the first liquid aerosol precursor in the first tank 412 comprises a nicotine formulation and the second liquid aerosol precursor in the second tank 414 comprises a flavour formulation, i.e., a nicotine free formulation.

The aerosol generating device further comprises an aerosol generation unit 416 in fluid communication with the first tank 412 and the second tank 414 such that the first liquid aerosol precursor is communicated from the first tank 412 to the aerosol generation unit 416 and such that the second liquid aerosol precursor is communicated from the second tank 414 to the aerosol generation unit 416.

In the example shown in Fig. 8, the aerosol generation unit 416 comprises a first aerosol generator 418 and a second aerosol generator 420, each of which comprises a piezoelectric transducer 100 and a drive circuit 300 as described above.

The first aerosol generator 418 is in fluid communication with the first tank 412 in order to communicate the first liquid aerosol precursor from the first tank 412 to the first aerosol generator 418, and in particular to the aerosolization surface of the piezoelectric transducer of the first aerosol generator 418, for example by way of a wick.

The second aerosol generator 420 is in fluid communication with the second tank 414 in order to communicate the second liquid aerosol precursor from the second tank 414 to the second aerosol generator 420, and in particular to the aerosolization surface of the piezoelectric transducer of the second aerosol generator 420, for example by way of a wick.

In the example shown in Fig. 8, the replaceable cartridge further comprises a cartridge memory unit 422 adapted to store a set of driving parameters of the aerosol generating unit 416. In particular, the cartridge memory unit 422 stores a first subset of one or more driving parameters for driving the first aerosol generator 418 and a second subset of one or more driving parameters for driving the second aerosol generator 420.

For driving the piezoelectric transducers of the first 418 and second 420 aerosol generators, the first and second subsets of one or more driving parameters comprise: a driving frequency; a driving duty cycle; and a driving power. The driving frequency for the first aerosol generator 418, i.e., the first driving frequency (for example 3MHz), may be higher than the driving frequency for the second aerosol generator 420, i.e., the second driving frequency (for example 1.5MHz).

In the example shown in Fig. 8, the replaceable cartridge 410 further comprises a cartridge communication unit 424 adapted to wirelessly communicate the set of one or more driving parameters from the cartridge memory unit 422 to a device communication unit 452 on the aerosol generating device 452 for driving the aerosol generating unit.

The aerosol generation unit 416, and in particular, the first aerosol generator 418 and the second aerosol generator 420 are communicatively linked, via a wired connection 453 between the replaceable cartridge 410 and the aerosol generating device 450, to a controller 454 adapted to generate a control signal for causing the drive circuit 546 to drive the aerosol generating unit 416 according to the set of one or more driving parameters received from the replaceable cartridge 410.

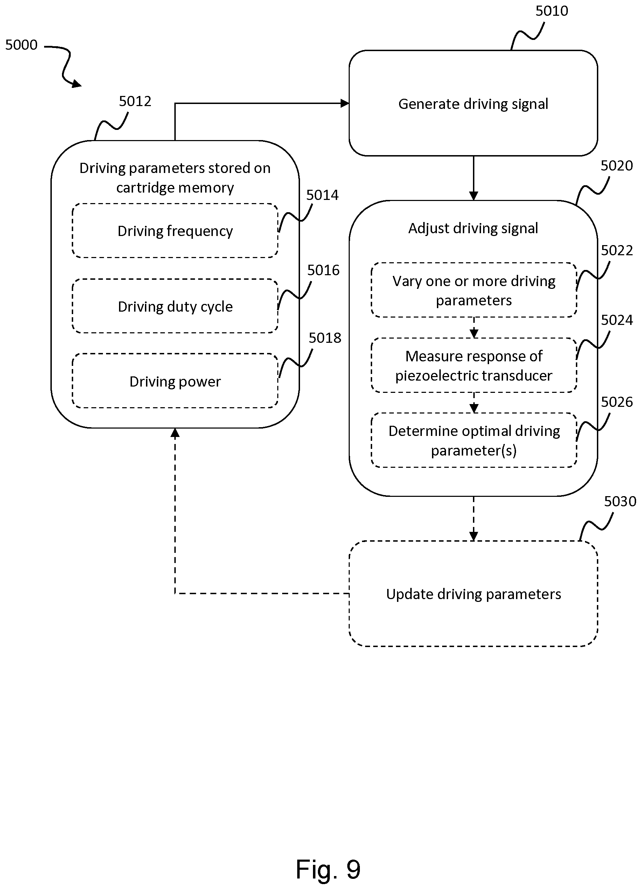

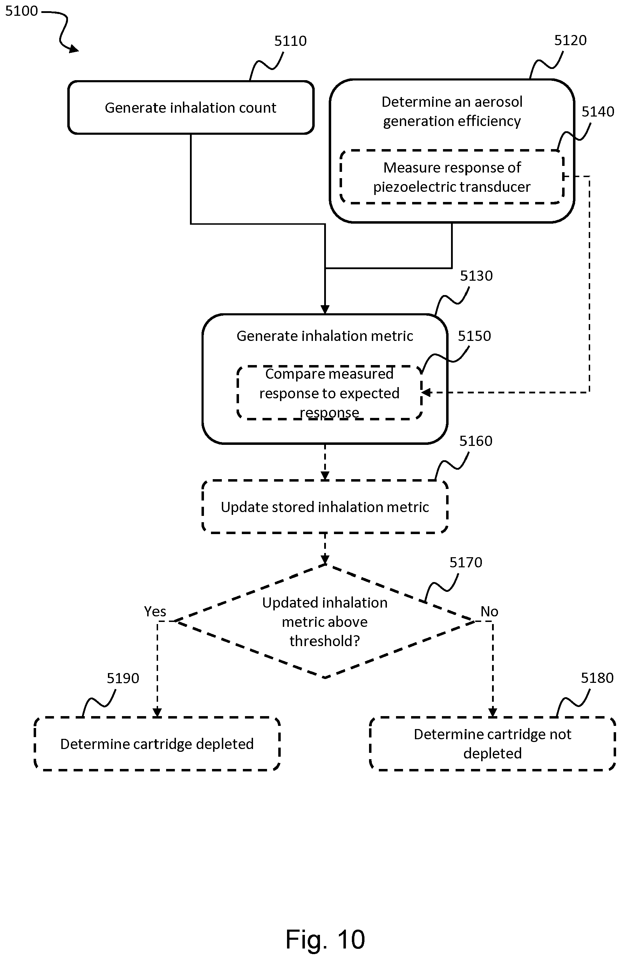

Fig. 9 shows a method 5000 for driving a piezoelectric transducer 100 of an aerosol generating system according to an aspect of the invention. The method shown in Fig. 10 may be performed for each piezoelectric transducer 100 of the aerosol generating system.

The method 5000 begins in step 5010 by generating a driving signal for driving the piezoelectric transducer 100 to produce aerosol from the liquid aerosol precursor. The driving signal is generated based on the set of one or more driving parameters 5012 stored on the cartridge memory unit including: driving frequency 5014; driving duty cycle 5016; and driving power 5018.

The method 5000 then progresses to step 5020, in which the driving signal is adjusted to change one or more of the driving parameters 5012 so as to produce a target response of the piezoelectric transducer to the driving signal.

The step of adjusting the driving parameters 5020 may include the sub-steps of varying 5022 the driving frequency, the driving duty cycle and/or the driving power and measuring 5024 the response of the piezoelectric transducer to the variation in each driving parameter.

In sub-step 5026, the optimal set of driving parameters, i.e., the optimal driving frequency, the optimal driving duty cycle and the optimal driving power are determined based on the measured response of the piezoelectric transducer to the variation in each driving parameter.

For example, the optimal driving parameters may include the determined resonant frequency of the piezoelectric transducer, the duty cycle that reduces complex frequency components in the piezoelectric transducer's response to the resonant frequency and the driving power that enables both the resonant frequency and duty cycle to be achieved. In a specific example, the optimal driving frequency may be 3MHz, the optimal duty cycle may be between 30% and 40%, and the optimal driving power may be 7.5 Watts.

The method may then progress to step 5030 where the optimal driving parameters determined in step 5026 are used to update the driving parameters 5012 stored on the cartridge memory unit to be used to generate the driving signal on the next inhalation of the user.

The method 4500 shown in Fig. 9 may be repeated with each inhalation of the user in order to track and update the optimal driving parameters for a piezoelectric transducer over the lifetime of the piezoelectric transducer.

Fig. 10 shows a method 5100 for controlling an aerosol generating system according to an aspect of the invention.

The method 5100 begins in step 5110 by generating an inhalation count, for example, by counting the number of activations of the aerosol generating unit in response to an inhalation of the user. Alongside step 5110, an aerosol generation efficiency is determined in step 5120 and then, in step 5130, the inhalation count and the aerosol generation efficiency are combined to generate the inhalation metric.

For example, in step 5140 the response of the piezoelectric transducer to the driving signal is measured and then compared, in step 5150, to the expected response of the piezoelectric transducer to a driving signal having the same driving parameters.

For example, for a single inhalation at 50% aerosol generation efficiency, the inhalation metric may be 0.5. In another example, for a series of three inhalations at an aerosol generation efficiency of 100%, the inhalation metric may be 3.

The method may then progress to step 5160 in which the inhalation metric stored on the cartridge memory unit is updated, for example by communication from the controller of the aerosol generating device, by way of the device communication unit.

In step 5170, the updated inhalation metric may then be compared to an inhalation metric threshold, which may be stored on the cartridge memory unit and specified at manufacture. If the updated inhalation metric is less than the inhalation metric threshold, the method progresses to step 5180 where it is determined that the replaceable cartridge is not depleted and may continue to be used. If the updated inhalation metric is greater than or equal to the inhalation metric threshold, the method progresses to step 5190, where it is determined that the replaceable cartridge is depleted and requires replacement.

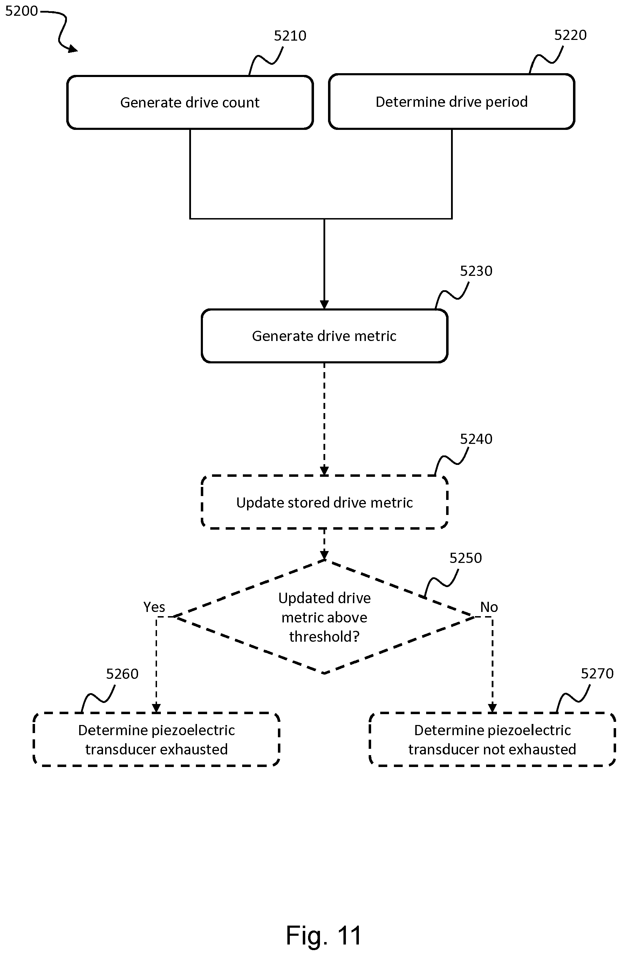

Fig. 11 shows a method 5200 for controlling an aerosol generating system according to an aspect of the invention.

The method 5200 begins in step 5210 by generating a drive count, for example, by counting the number of activations of the aerosol generating unit in response to an inhalation of the user. Alongside step 5210, a drive period is determined in step 5220 and then, in step 5230, the drive count and the drive period are combined to generate the drive metric.

For example, for a single inhalation lasting two seconds, the drive metric may be 2. In another example, for a series of three inhalations lasting one second each, the inhalation metric may be 3.

The method may then progress to step 5240 in which the drive metric stored on the cartridge memory unit is updated, for example by communication from the controller of the aerosol generating device, by way of the device communication unit.

In step 5250, the updated drive metric may then be compared to a drive metric threshold, which may be stored on the cartridge memory unit and specified at manufacture. If the updated drive metric is less than the drive metric threshold, the method progresses to step 5260 where it is determined that the piezoelectric transducer is not exhausted and may continue to be used. If the updated drive metric is greater than or equal to the inhalation metric threshold, the method progresses to step 5270, where it is determined that the piezoelectric transducer is exhausted and can no longer be used.

### REFERENCES

The entirety of the following documents, which are referenced in the present disclosure, are incorporated by reference into the present disclosure:
- Kooij, S., Astefanei, A., Corthals, G.L. etal. Size distributions of droplets produced by ultrasonic nebulizers. SciRep 9, 6128 (2019). https://doi.org/10.1038/s41598-019-42599-8

## Claims

1. A replaceable cartridge for an aerosol generating device, the replaceable cartridge comprising:
a tank for containing a liquid aerosol precursor;
an aerosol generating unit for generating an aerosol from the liquid aerosol precursor, wherein the aerosol generating unit comprises a piezoelectric transducer;
a cartridge memory unit adapted to store an inhalation metric associated with the aerosol generating unit; and
a cartridge communication unit adapted to:
receive an inhalation metric update from the aerosol generating device for updating the inhalation metric stored in the cartridge memory unit.

2. The replaceable cartridge as claimed in any preceding claim, wherein the tank comprises:
a first container for containing a first liquid aerosol precursor; and
a second container for containing a second liquid aerosol precursor; and
wherein the aerosol generating unit comprises:
a first aerosol generator for generating a first aerosol from the first liquid aerosol precursor; and
a second aerosol generator for generating a second aerosol from the second liquid aerosol precursor.

3. The replaceable cartridge as claimed in claim 2, wherein the first aerosol generator comprises a first piezoelectric transducer and the second aerosol generator comprises a second piezoelectric transducer.

4. The replaceable cartridge as claimed in any of claims 2 to 3, wherein the inhalation metric comprises a first inhalation metric associated with the first aerosol generator and a second inhalation metric associated with the second aerosol generator.

5. The replaceable cartridge claimed in any of claims 2 to 4, wherein the first liquid aerosol precursor comprises a nicotine formulation, and wherein the second liquid aerosol precursor comprises a flavour formulation.

6. The replaceable cartridge claimed in any preceding claim, wherein the cartridge memory unit is further adapted to store a driving metric associated with the piezoelectric transducer, and wherein the cartridge communication unit is further adapted to receive a driving metric update from the aerosol generating device for updating the driving metric stored in the cartridge memory unit.

7. An aerosol generating system comprising:
a replaceable cartridge according to any of claims 1 to 6; and
an aerosol generating device adapted to receive the replaceable cartridge, the aerosol generating device comprising:
a sensor for detecting an inhalation of the user;
a drive circuit for driving the aerosol generating unit according to a set of one or more driving parameters in response to a detected inhalation;
electrical circuitry adapted to:
generate an inhalation count based on a number of inhalations detected by the sensor;
monitor the driving of the aerosol generating unit to determine an aerosol generation efficiency for each inhalation; and
generate an inhalation metric update based on the inhalation count and the aerosol generation efficiency; and
a device communication unit adapted to communicate with the cartridge communication unit for communicating the inhalation metric update to the cartridge communication unit.

8. The aerosol generating system as claimed in claim 7, wherein monitoring the driving of the aerosol generating unit comprises measuring a response of the piezoelectric transducer to the set of one or more driving parameters for driving the piezoelectric transducer.

9. The aerosol generating system as claimed in any of claims 7 to 8, wherein generating the inhalation metric update comprises:
comparing the response of the piezoelectric transducer to the set of one or more driving parameters to an expected response of the piezoelectric transducer to the set of one or more driving parameters; and
generating the aerosol generation efficiency based on the comparison between the response of the piezoelectric transducer and the expected response of the piezoelectric transducer.

10. The aerosol generating system as claimed in any of claims 7 to 9, wherein the cartridge communication unit is further adapted to communicate an updated inhalation metric to the aerosol generating device, and wherein the electrical circuitry is further adapted to generate a control signal for adjusting a behaviour of the aerosol generating device.

11. The aerosol generating system as claimed in claim 10, wherein the electrical circuitry is further adapted to:
compare the updated inhalation metric to an inhalation metric threshold; and
if the updated inhalation metric is greater than or equal to the inhalation metric threshold, determine that the replaceable cartridge is depleted; and
if the updated inhalation metric is less than the inhalation metric threshold, determine that the replaceable cartridge is not depleted.

12. The aerosol generating system as claimed in claim 11, wherein, in response to determining that the replaceable cartridge is depleted, the electrical circuitry is further adapted to prevent the driving circuit from driving the piezoelectric transducer.

13. The aerosol generating system as claimed in any of claims 7 to 12, wherein the electrical circuitry is further adapted to:
generate a drive count based on a number of driving signals sent to the piezoelectric transducer;
monitor the driving of the aerosol generating unit to determine a driving period for each driving signal; and
generate the drive metric update based on the drive count and the aerosol driving period.

14. The aerosol generating system as claimed in claim 13, wherein the cartridge communication unit is further adapted to communicate an updated drive metric to the aerosol generating device, and wherein the electrical circuitry is further adapted to:
compare the updated drive metric to a drive metric threshold; and
if the updated drive metric is greater than or equal to the drive metric threshold, determine that the piezoelectric transducer is exhausted; and
if the updated drive metric is less than the drive metric threshold, determine that the piezoelectric transducer is not exhausted.

15. A computer-implemented method for use in an aerosol generating system according to any of claims 7 to 14, wherein the computer implemented method comprises:
generating an inhalation count based on a number of inhalations detected by the sensor;
monitoring the driving of the aerosol generating unit to determine an aerosol generation efficiency for each inhalation; and
generating an inhalation metric update based on the inhalation count and the aerosol generation efficiency.
